# EUROPEAN PATENT SPECIFICATION

(11) **EP 3 146 975 B1**
(45) Date of publication and mention of the grant of the patent: **02.10.2019**
(21) Application number: 15793248.4
(22) Date of filing: 14.05.2015
(51) Int. Cl.: A61K 35/747, A61P 31/04

(54) **AGENT FOR PREVENTION AND TREATMENT OF CHLAMYDIA INFECTION**
WIRKSTOFF ZUR PRÄVENTION ODER BEHANDLUNG EINER CHLAMYDIENINFEKTION
AGENT DE PRÉVENTION ET DE TRAITEMENT DE L'INFECTION À CHLAMYDIA

(30) Priority: 15.05.2014 JP 2014101200
(43) Date of publication of application: 29.03.2017
(73) Proprietor: Kabushiki Kaisha Yakult Honsha, Minato-ku Tokyo 105-8660 (JP)
(72) Inventor: ASAHARA Takashi, Tokyo 105-8660 (JP); TAKAHASHI Akira, Tokyo 105-8660 (JP); NOMOTO Koji, Tokyo 105-8660 (JP); KURAKAWA Takashi, Tokyo 105-8660 (JP); KADO Shoichi, Tokyo 105-8660 (JP); YUKI Norikatsu, Tokyo 105-8660 (JP); TAKAHASHI Takuya, Tokyo 105-8660 (JP)
(74) Representative: Blodig, Wolfgang
(86) International application number: PCT/JP2015/063858
(87) International publication number: WO 2015/174481

(56) References cited:
- EP-A1- 2 206 506
- CN-A- 102 120 045
- JP-A- 2002 080 364
- JP-A- 2012 512 828
- KELTZ M D ET AL: "Chlamydia serology screening in infertility patients", FERTILITY AND STERILITY, ELSEVIER SCIENCE INC, NEW YORK, NY, USA, vol. 85, no. 3, 1 March 2006 (2006-03-01), pages 752-754, XP028061819, ISSN: 0015-0282, DOI: 10.1016/J.FERTNSTERT.2005.08.029 [retrieved on 2006-03-01]
- MASAHIRO ITO ET AL.: 'Transposon mutagenesis of probiotic Lactobacillus casei identifies the asparagine synthetase gene asnH that contributes to activation of innate immunity' JOURNAL OF INTESTINAL MICROBIOLOGY vol. 27, no. 2, page PAGE 101, PAGE 101, XP055360756
- TAKASHI ASAHARA ET AL.: 'Tazai Taisei Salmonella enterica serovar Typhimurium DT104 Mouse Chokan Kansen Model ni Okeru, Kakushu Nyusan Kankin no Kansen Bogyo Sayo no Hikaku' JOURNAL OF THE JAPANESE ASSOCIATION FOR INFECTIOUS DISEASES vol. 78, no. 2, 2004, pages 193 - 194, XP008185640
- TAKASHI ASAHARA ET AL.: 'Shinki na Mouse Nyoro Daichokin Kansensho Jikken Model ni Okeru Nyusan Kankin Lactobacillus casei Shirota Kabu no Bokonai Chunyu ni yoru Kansen Bogyo Koka' JOURNAL OF THE JAPANESE ASSOCIATION FOR INFECTIOUS DISEASES vol. 74, no. 10, 2000, pages 874 - 875, XP008185645
- MASATO NAGAOKA ET AL.: 'Nyusan Kankin Lactobacillus casei YIT9018 Yurai no Tato - Peptide Glycan Fukugotai no Butsuri Kagakuteki Seijo' BASIC PHARMACOLOGY & THERAPEUTICS vol. 18, no. 1, 1990, pages 59 - 65, XP008185646
- KAN SHIDA: 'Anti-allergic activity of Lactobacillus casei YIT 9029 (Shirota strain): Action mechanisms and possible application in clinical practice' JOURNAL OF JAPAN SOCIETY FOR LACTIC ACID BACTERIA vol. 21, no. 2, 2010, pages 107 - 111, XP055360759
- DE WAARDA, R. ET AL.: 'Antagonistic activity of Lactobacillus casei strain Shirota against gastrointestinal Listeria monocytogenes infection in rats' INT. J. FOOD MICROBIOLOGY vol. 73, no. 1, 2002, pages 93 - 100, XP055360760
- TAKASHI ASAHARA ET AL.: 'Chlamydia Seishokuki Kansen Mouse Model ni Okeru Probiotics no Kansen Bogyo Koka' JAPANESE JOURNAL OF CHEMOTHERAPY vol. 62, no. SUPP.A, 19 May 2014, pages 321 , P 1 - 017, XP008185651

## Description

### Technical Field

The present invention relates to an agent for use in the prevention and treatment of chlamydia infection containing *Lactobacillus casei* as an active ingredient.

### Background Art

Chlamydia infection is a disease caused by the pharyngeal or genital infection with a microorganism of *Chlamydia.* In particular, the infection in women causes cervicitis, pelvic inflammatory disease or the like which may result in infertility due to oviduct disorder in severe cases.

Some patients with chlamydia infection have almost no subjective symptom, and women sometimes become pregnant without realizing chlamydia infection. Increase in the risk of abortion or premature birth in this case is a big social problem. Moreover, it is estimated that the number of patients with chlamydia infection increases especially among young people. Many of the patients do not receive any medical treatment when there is almost no subjective symptom, and it is a big social problem that the patients who do not realize the infection spread the infection by sex.

Various methods for preventing and treating chlamydia infection have been investigated so far, and for example, an antibacterial agent against a microorganism of *Chlamydia* has been reported (PTL 1) .

However, because microorganisms of *Chlamydia* are intracellular parasites, there is a problem in that persistent infection is easily caused and that an antibacterial agent is not so effective. Moreover, prolonged administration is preferable to completely kill microorganisms of *Chlamydia,* but antibacterial agents are often not suitable for prolonged administration because antibacterial agents generally have side effects.

### Citation List

### Patent Literature

PTL 1: JP-A-9-216824

CN 102120045 A discloses a probiotic-containing female hygienic product.

EP 2206506 A1 discloses probiotic formulations comprising live bacteria.

### Summary of Invention

### Technical Problem

Accordingly, a problem to be solved by the invention is to provide an agent for use in the prevention and treatment of chlamydia infection and an agent for use in the improvement of infertility caused by chlamydia infection which are highly safe and which can be administered over an extended period of time.

### Solution to Problem

As a result of intensive investigation to solve the above problem, the present inventors have found that chlamydia infection can be prevented and/or treated by administering *Lactobacillus casei.* The inventors have also found that infertility caused by chlamydia infection can be improved by administering *Lactobacillus casei.* The invention is defined by the appended claims.

That is, the invention relates in particular to an agent for use in the prevention and treatment of chlamydia infection, which is for oral administration and contains dead cells or pulverized cells of *Lactobacillus casei* as an active ingredient.

The invention also relates to an agent for use in the improvement of infertility caused by chlamydia infection, which is for oral administration and contains dead cells or pulverized cells of *Lactobacillus casei* as an active ingredient.

### Advantageous Effects of Invention

The agent for the prevention and treatment of chlamydia infection of the invention contains *Lactobacillus casei* as an active ingredient and is excellent because the agent can prevent and/or treat chlamydia infection through oral administration.

Moreover, the agent for the improvement of infertility caused by chlamydia of the invention contains *Lactobacillus casei* as an active ingredient and can improve infertility caused by chlamydia through oral administration. The term infertility in the invention includes both the inability to conceive even after a certain period of sex life with the desire to conceive and the inability of a fetus (embryo) to grow until the birth due to abortion or premature birth after conception. Thus, the agent for the improvement of infertility of the invention can improve the fertility rate of female individuals. In this regard, the fertility rate in the invention means the ratio of female individuals which have given birth normally to the female individuals which have been mated with male individuals.

### Brief Description of Drawings

[Fig. 1] A figure showing the counts of cells of the chlamydia bacterium in the vagina measured in Example 1.
[Fig. 2] A figure showing the fertility rates of mice calculated in Example 2.

### Description of Embodiments

*Lactobacillus casei* which serves as the active ingredient of the agent for use in the prevention and treatment of chlamydia infection and the agent for the use in improvement of infertility of the invention (hereinafter together referred to as "the preventive and therapeutic agent of the invention") is not particularly limited as long as it is classified as *Lactobacillus casei,* and those which are commercially available, those which have been deposited at a depositary authority such as ATCC, those which are newly found, mutants thereof and the like can be used. Examples of such *Lactobacillus casei* include *Lactobacillus casei* YIT9018, *Lactobacillus casei* YIT9029 and the like. Of the *Lactobacillus casei* strains, *Lactobacillus casei* YIT9029 is preferable. In this regard, the *Lactobacillus casei* strain does not have the capability of producing hydrogen peroxide and is highly safe.

*Lactobacillus casei* YIT9029 was deposited on May 18, 1987 at the Ministry of International Trade and Industry, the Agency of Industrial Science and Technology, the Fermentation Research Institute (the Fermentation Research Institute has become the current National Institute of Technology and Evaluation, the Patent Microorganisms Depositary and has been transferred to a new address Room 120, 2-5-8 Kazusakamatari, Kisarazu-shi, Chiba 292-0818) as FERM BP-1366. *Lactobacillus casei* YIT9018 was deposited on November 14, 1984 at the Ministry of International Trade and Industry, the Agency of Industrial Science and Technology, the Fermentation Research Institute (the Fermentation Research Institute has become the current National Institute of Technology and Evaluation, the Patent Microorganisms Depositary and has been transferred to a new address Room 120, 2-5-8 Kazusakamatari, Kisarazu-shi, Chiba 292-0818) as FERM BP-665.

The form of *Lactobacillus casei in* the preventive and therapeutic agent of the invention is dead bacterial cells, or pulverized bacterial cells. The *Lactobacillus casei* content of the preventive and therapeutic agent of the invention is not particularly limited and may be an amount corresponding to the dosage form described below. In the case of dead cells, live cells of 1×10⁷ to 1×10¹³ CFU can be killed by heating or the like. The administration time is not particularly limited, and the agent can be administered before the infection for the purpose of prevention or administered after the infection for the purpose of treatment. However, because the risk of abortion or premature birth increases in the case of conception after the infection with chlamydia, it is preferable to administer the preventive and therapeutic agent of the invention before the conception and before the infection for the purpose of prevention. The administration period is not particularly limited, but the agent is preferably administered continuously from seven days or more before the infection when the purpose is prevention. Also, when the purpose is treatment, the agent is preferably administered continuously for three days or more after the infection. The upper limit of the administration period is not particularly limited, but an example is continuous administration for one to two weeks. The administration frequency per day is not particularly limited, either, but an example thereof is once to three times a day. When the agent is administered as the agent for the improvement of infertility, the agent can be administered in the same manner as in the case for the purpose of prevention and treatment.

The preventive and therapeutic agent of the invention should contain *Lactobacillus casei* as an active ingredient. Thus, the agent may consist of *Lactobacillus casei* or may be composed as a combination with a pharmaceutically acceptable carrier.

Examples of the pharmaceutically acceptable carrier include glucose, lactose, sucrose, starch, mannitol, dextrin, fatty acid glycerides, polyethylene glycol, hydroxyethyl starch, ethylene glycol, polyoxyethylene sorbitan fatty acid esters, amino acids, gelatin, albumin, water, a physiological saline solution and the like. According to the need, a generally used additive such as a stabilizer, a wetting agent, an emulsifier, a binder, a tonicity agent and an excipient can be suitably added.

The dosage form of the preventive and therapeutic agent of the invention is not particularly limited, but examples thereof include liquid, powder, granules, capsules, tablets, suppositories, external preparations and the like. The preventive and therapeutic agent of the invention in such a dosage form can be prepared according to a known method for preparing a preparation using a lactic acid bacterium.

The preventive and therapeutic agent of the invention obtained in this manner can be used for the prevention and/or treatment of chlamydia infection caused by a microorganism of *Chlamydia* such as *Chlamydia trachomatis* or *Chlamydia muridarum.*

Moreover, the agent for the improvement of infertility of the invention can improve infertility caused by chlamydia infection and improve the fertility rate.

In this regard, the preventive and therapeutic agent of the invention exerts the effects through oral administration. Oral administration is easy and suitable for prolonged administration.

By adding a predetermined amount of *Lactobacillus casei* to a food, a food exhibiting the action of preventing and treating chlamydia infection can be produced. That is, *Lactobacillus casei* is used as a food additive (material) used for giving the action of preventing and treating chlamydia infection to a final product, namely a food additive composition for the prevention and/or treatment of chlamydia infection containing *Lactobacillus casei.* The form of *Lactobacillus casei* in the food additive composition of the invention is dead bacterial cells, or pulverized bacterial cells. The *Lactobacillus casei* content of the final product to which the food additive composition of the invention has been added is not particularly limited. In the case of dead cells, live cells of 1×10⁷ to 1×10¹³ CFU can be killed by heating or the like.

### Examples

### Production Example 1

### Production of Solutions of Lactobacillus casei Cells:

*Lactobacillus casei* YIT9029 (hereinafter referred to as "LcS") was cultured in 10 ml of MRS medium at 37°C for 24 hours and then centrifugally washed twice under the conditions of at 4°C at 5,000 g for five minutes with a sucrose-phosphate gultamate buffer (hereinafter referred to as "the SPG buffer" : 75 g/L sucrose, 0.72 g/L L-glutamic acid, 6.148 g/L disodium hydrogen phosphate 12H₂O and 0.247 g/L potassium dihydrogen phosphate in distilled water, pH 7.0). The supernatant was removed, and the cells were suspended again in 1 ml and 10 ml of the SPG buffer to produce LcS cell solutions. The LcS cell solution obtained by suspending the cells again in 1 ml of the SPG buffer was used for transvaginal administration (live LcS cell count of 1×10¹⁰ CFU/ml), and the LcS cell solution obtained by suspending the cells again in 10 ml of the SPG buffer was used for oral administration (live LcS cell count of 1×10⁹ CFU/ml) . The LcS cell solutions for transvaginal administration and oral administration were heated at 100°C for 30 minutes to produce heated dead cell solutions.

### Reference Production Example 1

### Production of Solutions of Lactobacillus johnsonii Cells

Lj cell solutions were produced in the same manner as in Production Example 1 except that *Lactobacillus johnsonii* YIT0219^{T} (a standard *Lactobacillus johnsonii* strain: hereinafter referred to as "Lj") was used instead of LcS.

### Example 1

### Prevention/Treatment of Chlamydia Infection by Administration of LcS

### (1) Preparation of Bacterium for Infection

A suspension of McCoy cells (4×10⁵ cells/ml) cultured in DMEM medium containing 10% fetal calf serum was dispensed to a 24 well plate at 1 ml per well, and the cells were cultured in a CO₂ incubator at 37°C for 24 hours. The cultured cells were infected with 1×10⁵ cells of *Chlamydia muridarum* ATCC VR123^{T} (hereinafter referred to as "MoPn") per 1 ml cultured cells, and then the supernatant was removed by centrifugation. Then, DMEM medium to which cycloheximide had been added at 1 µg/ml was added, and the cells were cultured in a CO₂ incubator at 37°C for 72 hours. The precipitates obtained by removing the MoPn-infected cells from the plate and centrifugation were suspended in the SPG buffer. The MoPn-infected cells were subjected to ultrasound treatment (28 kHz, 5 seconds, 10 times), and the supernatant was collected by centrifugation. The MoPn in the supernatant was centrifugally washed twice under the conditions of at 4°C at 15,000 g for an hour with the SPG buffer and then suspended in the SPG buffer to produce a solution for infection (1×10⁸ cells/ml).

### (2) Infection of Mice with MoPn

To regulate the estrous cycle of female mice, progesterone (luteum injection: ASKA Pharmaceutical Co., Ltd.) was subcutaneously administered at 2.5 mg per mouse. The mice whose estrous cycle had been regulated were anesthetized with Somnopentyl according to a general method. Ten microliters (MoPn cell count of 1×10⁵ cells) of the solution for infection were injected into the vagina of each mouse under anesthesia to infect the mouse with MoPn.

### (3) Administration of Cell Solutions (Six Mice Per Group)

### [Group with Live LcS Cell Transvaginal Administration]

Under anesthesia with Somnopentyl, 10 µl (1×10⁸ CFU) of the LcS cell solution for transvaginal administration produced in Production Example 1 was administered to each mouse every day from seven days before the infection to three days after the infection once a day in the vagina.

### [Group with Dead LcS Cell Transvaginal Administration]

Under anesthesia with Somnopentyl, 10 µl of the heated dead LcS cell solution for transvaginal administration produced in Production Example 1 was administered to each mouse every day from seven days before the infection to three days after the infection once a day in the vagina. Ten microliters of the heated dead LcS cell solution correspond to a live LcS cell count of 1×10⁸ CFU before heating.

### [Group with Live Lj Cell Transvaginal Administration]

Under anesthesia with Somnopentyl, 10 µl (1×10⁸ CFU) of the Lj cell solution for transvaginal administration produced in Reference Production Example 1 was administered to each mouse every day from seven days before the infection to three days after the infection once a day in the vagina.

### [Control Group with Transvaginal Administration]

Under anesthesia with Somnopentyl, 10 µl of the SPG buffer was transvaginally administered to each mouse every day from seven days before the infection to three days after the infection once a day.

### [Group with Live LcS Cell Oral Administration]

A mouse was fixed with fingers, and 100 µl (1×10⁸ CFU) of the LcS cell solution for oral administration produced in Production Example 1 was orally administered to the mouse using a stomach tube every day from seven days before the infection to three days after the infection once a day.

### [Group with Dead LcS Cell Oral Administration]

A mouse was fixed with fingers, and 100 µl of the heated dead LcS cell solution for oral administration produced in Production Example 1 was orally administered to the mouse using a stomach tube every day from seven days before the infection to three days after the infection once a day. A hundred microliters of the heated dead LcS cell solution correspond to a live LcS cell count of 1×10⁸ CFU before heating.

### [Group with Live Lj Cell Oral Administration]

A mouse was fixed with fingers, and 100 µl (1×10⁸ CFU) of the Lj cell solution for oral administration produced in Reference Production Example 1 was orally administered to the mouse using a stomach tube every day from seven days before the infection to three days after the infection once a day.

### [Control Group with Oral Administration]

A mouse was fixed with fingers, and 100 µl of the SPG buffer was orally administered to the mouse using a stomach tube every day from seven days before the infection to three days after the infection once a day.

### (4) Counting of MoPn Cells

On the third day after the infection with MoPn, the vaginal tissue was collected aseptically from each mouse which had been slaughtered by bleeding under anesthesia with Somnopentyl according to a general method, and the tissue was floated on the SPG buffer and then cryopreserved at -80°C. The tissue was thawed at room temperature and homogenized, and then 200 µl of the homogenate was put into a tube containing 400 µl of RNAprotect (for quantitative RT-PCR: Qiagen) to extract the RNA. Then, the measurement was conducted by the quantitative RT-PCR method using Qiagen Onestep RT-PCR kit and using *Chlamydia trachomatis-* and *Chlamydia muridarum-specific* primers. More specifically, reverse transcription was conducted at 50°C for 30 minutes and at 95°C for 15 minutes, and 45 cycles each consisting of 20 seconds at 94°C, 20 seconds at 55°C and 50 seconds at 72°C were conducted. The nucleotide sequences of the *Chlamydia trachomatis-* and *Chlamydia muridarum-specific* primers were the forward primer: TGCATAGATAATTTGTCCTTAACTTG (SEQ ID NO: 1) and the reverse primer: CGAGATTTGACAACTAACTTACCT (SEQ ID NO: 2).

### (5) Statistical Analysis

The average MoPn cell count and the standard deviation of each group were calculated. The statistical significance of an administration group compared to the control group was tested by the non-parametric Mann-Whitney's U test, and the statistical difference between groups was tested by the multiple comparison method (Bonferroni). SPSS Ver.11 (SPSS Inc.) was used as the software for the statistical analysis, and the significance level was 5% in a two-tailed test in both tests.

### (6) Results

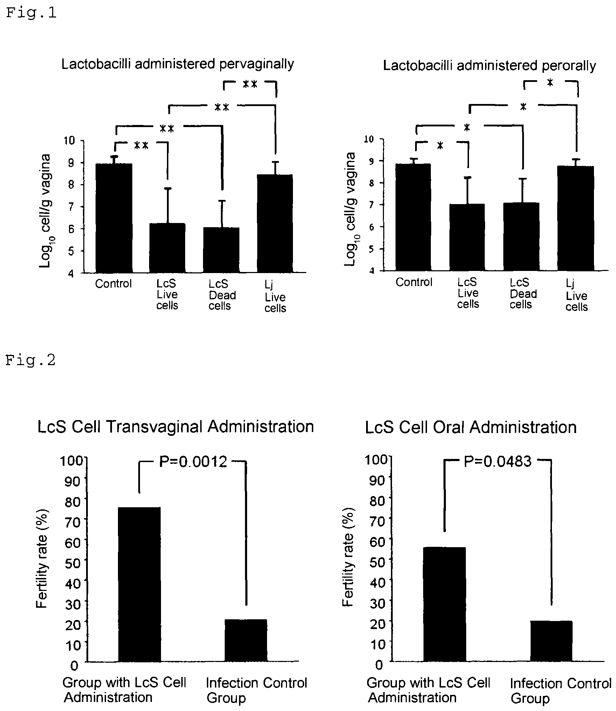

The results of the MoPn cell counts in the vagina are shown in Fig. 1.

In the group with live LcS cell transvaginal administration, the MoPn cell count in the vagina was significantly lower (P<0.01) than that of the control group with transvaginal administration. Also in the group with dead LcS cell transvaginal administration, the action of protecting against the infection which was as significant as that of the group with live LcS cell transvaginal administration was observed (P<0. 01). On the other hand, the action of protecting against the vaginal infection with MoPn was not observed in the group with live Lj cell transvaginal administration. In Fig. 1, ∗∗ indicates P<0.01.

Also in the group with live LcS cell oral administration, the MoPn cell count in the vagina was significantly lower (P<0.05) than that of the control group with oral administration, and the action of protecting against the vaginal infection with MoPn was observed. Moreover, in the group with dead LcS cell oral administration, the action of protecting against the infection which was as significant as that of the group with live LcS cell oral administration was observed (P<0. 05) . On the other hand, the action of protecting against the vaginal infection with MoPn was not observed in the group with live Lj cell oral administration. In Fig. 1, * indicates P<0.05.

### Example 2

### Improvement of Fertility Rate of Mice by LcS Administration:

### (1) Infection of Mice with MoPn

To regulate the estrous cycle of female mice, progesterone (luteum injection: ASKA Pharmaceutical Co., Ltd.) was subcutaneously administered at 2.5 mg per mouse. The mice whose estrous cycle had been regulated were anesthetized with Somnopentyl according to a general method. Ten microliters (MoPn cell count of 1×10⁶ cells) of the solution for infection prepared in Example 1 were injected into the vagina of each mouse under anesthesia to infect the mouse with MoPn.

### (2) Administration of Cell Solutions (20 Mice Per Group)

### [Group with Live LcS Cell Transvaginal Administration]

Under anesthesia with Somnopentyl, 10 µl (1×10⁸ CFU) of the LcS cell solution for transvaginal administration produced in Production Example 1 was administered to each mouse every day from seven days before the infection to three days after the infection once a day in the vagina.

### [Control Group with Transvaginal Administration]

Under anesthesia with Somnopentyl, 10 µl of the SPG buffer was transvaginally administered to each mouse every day from seven days before the infection to three days after the infection once a day.

### [Group with Live LcS Cell Oral Administration]

A mouse was fixed with fingers, and 100 µl (1×10⁸ CFU) of the LcS cell solution for oral administration produced in Production Example 1 was orally administered to the mouse using a stomach tube every day from seven days before the infection to three days after the infection once a day.

### [Control Group with Oral Administration]

A mouse was fixed with fingers, and 100 µl of the SPG buffer was orally administered to the mouse using a stomach tube every day from seven days before the infection to three days after the infection once a day.

### (3) Mating of Mice

A female mouse on the third day after the infection with MoPn was raised in a cage with a male mouse at male 1:female 1 for five days, thereby mating the female mouse with the uninfected male mouse. A plug (a white waxy substance attached to the vaginal opening of a female), which is an indicator of pregnancy, was checked every morning and evening from the following day of mating to the end of mating. When the plug was observed, the female individual and the male individual were separated, and whether or not the female mouse gave birth was observed under the raising condition of one mouse per case until the 32nd day after the infection.

### (4) Statistical Analysis

The fertility rates were tested by the Fisher's exact test. SPSS Ver.11 (SPSS Inc.) was used as the software for the statistical analysis, and the significance level was 5% in a two-tailed test.

### (5) Results

The fertility rates calculated from the presence or absence of birth of the mice are shown in Fig. 2. The fertility rates of the infection control groups were 20% (four out of 20 mice) . On the other hand, the fertility rate of the group with LcS transvaginal administration was 75% (15 out of 20 examples), and a significant action of improving the infertility of the mice infected with MoPn was observed (P=0.0012). Moreover, also in the group with LcS oral administration, a significant action of improving the infertility of the mice infected with MoPn was observed (the fertility rate was 55% [11 out of 20 examples], P=0.0483).

### Example 3

### Change of Chlamydia Cell Count by LcS Administration

### (1) Infection of Mice with MoPn

To regulate the estrous cycle of female mice, progesterone (luteum injection: ASKA Pharmaceutical Co., Ltd.) was subcutaneously administered at 2 . 5 mg per mouse . The mice whose estrous cycle had been regulated were anesthetized with Somnopentyl according to a general method. Ten microliters (MoPn cell count of 1×10⁵ cells) of the solution for infection prepared in Example 1 were injected into the vagina of each mouse under anesthesia to infect the mouse with MoPn.

### (2) Administration of Cell Solutions (Six Mice Per Group)

### [Group with Live LcS Cell Transvaginal Administration]

Under anesthesia with Somnopentyl, 10 µl (1×10⁸ CFU) of the LcS cell solution for transvaginal administration produced in Production Example 1 was administered to each mouse every day from seven days before the infection to three days after the infection once a day in the vagina.

### [Control Group with Transvaginal Administration]

Under anesthesia with Somnopentyl, 10 µl of the SPG buffer was transvaginally administered to each mouse every day from seven days before the infection to three days after the infection once a day.

### [Group with Live LcS Cell Oral Administration]

A mouse was fixed with fingers, and 100 µl (1×10⁸ CFU) of the LcS cell solution for oral administration produced in Production Example 1 was orally administered to the mouse using a stomach tube every day from seven days before the infection to three days after the infection once a day.

### [Control Group with Oral Administration]

A mouse was fixed with fingers, and 100 µl of the SPG buffer was orally administered to the mouse using a stomach tube every day from seven days before the infection to three days after the infection once a day.

### (3) Counting of MoPn Cells

By the same method as in Example 1, the vaginal tissue was collected from the mice on the 21st day after the infection with MoPn, and the MoPn cells in the vagina were counted.

### (4) Results

In the control group with transvaginal administration, 1×10⁵ cells/g of MoPn was detected from three of the six mice also on the 21st day after the infection. On the other hand, in the group with live LcS cell transvaginal administration, the administration of the LcS cell solution had a continuous effect even though the administration was finished on the third day after the infection, and MoPn was detected from none of the mice on the 21st day after the infection.

Moreover, in the control group with oral administration, 1×10⁵ cells/g of MoPn was detected from three of the six mice also on the 21st day after the infection. On the other hand, in the group with live LcS cell oral administration, the administration of the LcS cell solution had a continuous effect even though the administration was finished on the third day after the infection, and MoPn was not detected from five of the six mice on the 21st day after the infection.

## Claims

1. An agent for use in the prevention and treatment of chlamydia infection, which is for oral administration and contains dead cells or pulverized cells of *Lactobacillus casei* as an active ingredient.

2. The agent for use in the prevention and treatment of chlamydia infection according to claim 1, wherein *Lactobacillus casei* is *Lactobacillus casei* YIT9029 (FERM BP-1366).

3. An agent for use in the improvement of infertility caused by chlamydia infection, which is for oral administration and contains dead cells or pulverized cells of *Lactobacillus casei* as an active ingredient.

4. The agent for use in the improvement of infertility according to claim 3, wherein *Lactobacillus casei* is *Lactobacillus casei* YIT9029 (FERM BP-1366).

## Patentansprüche

1. Mittel zur Verwendung bei der Prävention oder Behandlung einer Chlamydieninfektion, das für die orale Verabreichung vorgesehen ist und tote Zellen oder pulverisierte Zellen von *Lactobacillus casei* als aktiven Inhaltsstoff enthält.

2. Das Mittel zur Verwendung bei der Prävention oder Behandlung einer Chlamydieninfektion gemäß Anspruch 1, wobei *Lactobacillus casei Lactobacillus casei* YIT9029 (FERM BP-1366) ist.

3. Mittel zur Verwendung bei der Verbesserung einer von einer Chlamydieninfektion verursachten Unfruchtbarkeit, das für die orale Verabreichung vorgesehen ist und tote Zellen oder pulverisierte Zellen von *Lactobacillus casei* als aktiven Inhaltsstoff enthält.

4. Das Mittel zur Verwendung bei der Verbesserung einer Unfruchtbarkeit gemäß Anspruch 3, wobei *Lactobacillus casei Lactobacillus casei* YIT9029 (FERM BP-1366) ist.

## Revendications

1. Agent pour une utilisation dans la prévention et le traitement d'une infection à chlamydia, qui est destiné à une administration orale et qui contient des cellules mortes ou des cellules pulvérisées de *Lactobacillus casei* en tant que principe actif.

2. Agent pour une utilisation dans la prévention et le traitement d'une infection à chlamydia selon la revendication 1, dans lequel *Lactobacillus casei* et *Lactobacillus casei* YIT9029 (FERM BP-1366).

3. Agent pour une utilisation dans l'amélioration d'une infécondité due à une infection à chlamydia, qui est destiné à une administration orale et qui contient des cellules mortes ou des cellules pulvérisées de *Lactobacillus casei* en tant que principe actif.

4. Agent pour une utilisation dans l'amélioration d'une infécondité selon la revendication 3, dans lequel *Lactobacillus casei* et *Lactobacillus casei* YIT9029 (FERM BP-1366) .


### SEQUENCE LISTING

<110> Kabushiki Kaisha Yakult Honsha
<120> MEDICINE FOR PROPHYLAXIS AND TREATMENT OF CHLAMYDIAL INFECTION
<130> PF-150002-WO
<150> JP2014-101200
   <151> 2014-05-15
<160> 2
<170> PatentIn version 3.5
<210> 1
   <211> 26
   <212> DNA
   <213> Artificial Sequence
<220>
   <223> Forward Primer
<400> 1
   tgcatagata atttgtcctt aacttg 26
<210> 2
   <211> 24
   <212> DNA
   <213> Artificial Sequence
<220>
   <223> Reverse Primer
<400> 2
   cgagatttga caactaactt acct 24